# EUROPEAN PATENT SPECIFICATION

(11) **EP 0 646 104 B1**
(45) Date of publication and mention of the grant of the patent: **09.10.1996**
(21) Application number: 93911386.6
(22) Date of filing: 24.05.1993
(51) Int. Cl.: C07C 15/08, C07C 7/148

(54) **PROCESS FOR IMPROVING PURITY OF $i(PARA)-XYLENE PRODUCT**
VERFAHREN ZUR VERBESSERUNG DER REINHEIT VON PARA-XYLOL
PROCEDE D'AMELIORATION DE LA PURETE DE $i(PARA)-XYLENE

(30) Priority: 26.05.1992 US 888577
(43) Date of publication of application: 05.04.1995
(73) Proprietor: EXXON CHEMICAL PATENTS INC., Baytown, TX 77520-2149 (US)
(72) Inventor: OU, John, Di-Yi, Houston, TX 77062 (US); HELMKE, Harold, William, Jr., Kingwood, TX 77339 (US); PILLIOD, Dana, Lynn, League City, TX 77573 (US)
(74) Representative: Bawden, Peter Charles
(86) International application number: US9304879
(87) International publication number: WO9324432

(56) References cited:
- GB-A- 1 108 178
- US-A- 2 801 271
- US-A- 2 816 940
- US-A- 2 840 621

## Description

### Field of the Invention

The invention relates to methods for producing *para*-xylene, and more particularly, the invention relates, in one aspect, to processes for separating *para*-xylene in exceptionally high purity from its isomers.

### Backaround of the Invention

There exist several technologies for recovering *para*-xylene from its mixture with other xylene isomers including *ortho*-xylene*, meta*-xylene and ethylbenzene. Adsorption-based methods and crystallization-based processes are commonly used in industry. These two processes can produce 99.6 to 99.8 wt.% purity *para*-xylene very efficiently. Achieving purities on the order of 99.9+ wt.%, however, is very difficult and costly. Other techniques for xylene separation such as hydrogen-fluoride extraction, sulfonation and alkylation have not been able to demonstrate an efficiency comparable to that of the adsorption and crystallization processes.

The chemistry for xylenes alkylation has been studied for several decades. D. Nightingale, et al. in "Orientation Effects in the Alkylation of *m*-Xylene by Various Procedures and Reagents," *Journal of the American Chemical Society,* Vol. 64, pp. 1662-1665, 1942; and D. Nightingale, et al. in "The Alkylation of *o*- and *p*-Xylene," *Journal of the American Chemical Society,* Vol. 66, pp. 154-155, 1944, indicate that *ortho*-xylene, *meta*-xylene and ethylbenzene can be alkylated with molecules containing tertiary butyl moiety (*e.g.* isobutylene, tertiary butyl chloride, tertiary butyl benzene, di-tertiary butyl hydroxyl toluene, etc.). *Para*-Xylene, however, was found to be quite difficult for tertiary butylation. It is theorized that a steric effect has hindered the insertion of the bulky tertiary butyl group to the more restricted aromatic ring of the *para*-xylene molecule. Partial separation of *para*-xylene from *meta*-xylene and *ortho*-xylene based on the selective alkylation concept has been demonstrated; see, *e.g.,* US-A-2,648,713; US-A-2,801,271 and B. B. Corson, et al., *Industrial and Engineering Chemistry,* Vol. 48, No. 7, pp. 1180, 1956.

US-A-2,648,713 provides a process for the separation of *ortho*-xylene from an admixture thereof with *meta*-xylenes by the successive steps of alkylation, distillation and dealkylation. *Para*-Xylene may also be present in the xylene mixture and remains unalkylated with the *meta*-xylene. The alkylating agent is preferably an olefin or cycloolefin having a tertiary carbon atom, such as isobutylene; diisobutylene; trimethylethylene; 2,4-dimethylpentene-2; 3-methylbutene-2; 4-methylcyclohexene-1 and 1-methylcyclohexene-1. This process, as the ones previously discussed, is limited to bulk separation; in the best Example recorded therein (Example 2) the percent conversion of ethyl benzene and *ortho*-xylene was 89 and 54, respectively.

US-A-2,801,271 describes an integrated process for separating xylene isomers and producing high octane gasoline which involves contacting isobutylene with a xylene fraction having substantial quantities of *para*-xylene and *meta*-xylene. The contact is done in the presence of liquid hydrogen fluoride at a temperature in the range -10 to 100°C. The quantity of isobutylene employed is sufficient to alkylate a substantial proportion of the *meta*-xylene contained in the xylene fraction. The alkylation reaction product is fractionally distilled to separate a *para*-xylene rich fraction and a fraction comprising *tertiary*butyl-*meta*-xylene. The latter fraction and a straight run petroleum distillate are contacted with a silica-alumina catalyst in a catalytic cracking zone at a temperature in the range 426 to 538°C (800 to 1000°F). The latter fraction is cracked forming predominantly *meta*-xylene and isobutylene. The effluent from the cracking zone is fractionally distilled to separate a fraction rich in isobutylene, and the isobutylene rich fraction is then returned together with additional xylenes to the alkylation step. Again, this process is a bulk separation process; the only example (Example 1) to the alkylation separation step yielded a *para*-xylene purity of 48%.

Purified *meta*-xylene is obtained by alkylating a mixture containing *m*- and *p*-xylene with a small but effective amount of isopropylating agent in the presence of a small but sufficient amount of aluminum chloride catalyst, according to US-A-3,539,650. The alkylation mixture is maintained at a temperature within the range of about 70°C to about 100°C for about thirty minutes to one hour after which the catalyst is destroyed, an isopropyl *meta*-xylene fraction recovered, and the recovered fraction deisopropylated to produce *meta*-xylene above 95% purity. It is apparent that *meta*-xylene concentrations much above 98% may not be readily attainable, however, given the best Examples in Table IV of this patent, which focuses only on the *meta*-xylene product, rather than the *para*-xylene material.

US-A-5,055,630 describes a process for obtaining a *para*-xylene final product more than 98% pure from a crystalline starting material with a purity of about 98%, which includes the steps of intermixing the starting material with precooled water and feeding back the recovered *para*-xylene in a mixer at a temperature of 0 to 13°C to form a *para*-xylene-crystal-water mixture containing *para*-xylene crystals and water. The mixture is continuously transferred as soon as it contains 30% by weight *para*-xylene crystals into a purifying centrifuge via a dewatering filter to form a fluid phase and a *para*-xylene crystal slurry. The fluid phase is separated further from the *para*-xylene crystals in a first stage of a purifying centrifuge, mixing the *para*-xylene crystal slurry in a second stage with a partial flow of final product, heating at about 13°C and subsequently liberating from the fluid phase still adhering, whereby the *para*-xylene crystals are drawn off into the heated vessel. Next, the *para*-xylene crystals melted in the heated vessel are drawn off as the final product with the desired purity. A partial flow of final product is also drawn off which is fed back to the second stage of the purifying centrifuge, being heated previously to a temperature from 60 to 80°C. and feeding the fluid phase separated from the *para*-xylene crystals to the second stage of the centrifuge for recovery of additional *para*-xylene which becomes part of the final product stream. It would be advantageous if a method could be devised for obtaining high purity *para*-xylene which was less complex and costly than that described in US-A-5,055,630.

The separation of close-boiling *meta*-xylene and *para*-xylene via selective alkylation and subsequent dealkylation or transalkylation was recently studied according to *Chemical Abstracts* 116(6):43394n (1992). The alkylation was carried out with isobutylene or diisobutylene with concentrated H₂SO₄ catalyst at -10 to +20°C and Filtrol-24 acid clay at 80-130°C, respectively. *Meta*-Xylene reacted very selectively (Filtrol-24 gave selectively 5-tert-butyl-*meta*-xylene), and the alkylated products could be dealkylated at higher temperatures in the presence of Filtrol-24 catalyst, to give relatively pure *meta*-xylene and isobutylene which could be recycled. Para-Xylene purity is not mentioned in the Abstract.

GB-A-1 108 178 describes a process for separating xylene isomers from a mixture containing *para*-xylene and at least one other xylene isomer, comprising treating the mixture with an xylene aromatic compound having an alkyl group of at least 3 carbon atoms in the presence of an alkylating catalyst to form alkyl adducts of ortho xylene and/or meta xylene and separating the *para*-xylene by distillation.

The Example of the patent using a starting mixture of equivalent weights of meta and *para*-xylene. The purity of the *para*-xylene obtained is not recorded.

There remains a need for a process which provides a high yield to *para*-xylene in very high purity which is relatively simple and cost-effective. The linking of two conventional *para*-xylene purification processes together is generally less efficient. Additionally, the reaction to make *para*-xylene is reversible under many of the conditions to selectively alkylate the xylene mixture, often in the attempt to make very high purity *para*-xylene, an isomerization side reaction occurs to make more of the less desirable *meta*-xylene and/or *ortho*-xylene. At relatively high purities, e.g. 98 wt %, attempts to increase purity of the already pure product result in degradation of the product rather than improvement through these side reactions. Also, *para*-xylene does alkylate to some extent and there is a great tendency when 98 wt % of the product is *para*-xylene that the alkylation will more readily occur with the *para*-xylene, rather than with the small proportion of impurities which are more difficult for the catalyst to come into contact with. Attempts often result in removing alkylated *para*-xylene and/or isomerizing *para*-xylene with the overall result of a relatively increased proportions of impurity and a product with a lowered purity to *para*-xylene.

Many of the prior processes do not discuss the problems of side reactions and reversible reactions.

Most of them are concerned only with bulk separations and are satisfied with conversions of about 50%. As discussed, only US-A-5,055,630 attempts to achieve high purity *para*-xylene, and it is only accomplished via a complicated crystallization process involving a multi-stage centrifuge. It is anticipated that in the near future there will be a great need for ultra-pure *para*-xylene, on the order of 99.9+ wt.% purity.

### Summary of the Invention

According to the present invention there is provided a process for producing at least 99.5 wt% *para*-xylene from a mixture of xylene isomers comprising the steps of:
(1) providing, as an intermediate product, a mixture of xylene isomers containing 99 to 99.8 wt% *para*-xylene; and
(2) recovering *para*-xylene from the intermediate product to produce a final product having *para*-xylene in a concentration which is at least 99.5 wt% and greater than the concentration of *para*-xylene in the intermediate product, by a second step which comprises:
   (a) alkylating substantially all of the xylene isomers in the intermediate product other than *para*-xylene with an alkylating agent having at least one tertiary butyl moiety in the presence of an acidic catalyst and will good molecular contact between the catalyst and the xylene isomers, while alkylating substantially none of the *para*-xylene; and
   (b) separating from the alkylated xylene isomers, *para*-xylene in a purity which is at least 99.5 wt.% and greater than the concentration of *para*-xylene in the intermediate product, to form the final product.

The process, preferably used continuously, permits production of *para*-xylene in very high purity by selective alkylation in a straight-forward, simple manner, generally with extremely high selectivity and little or no reverse reactions and side reactions.

In one embodiment of the invention, there is provided a technique for producing at least 99.5 wt.% *para*-xylene from a mixture of xylene isomers by first recovering *para*-xylene from a mixture of xylene isomers by a first step (typically using conventional processes) where the concentration of *para*-xylene in an intermediate product or stream ranges up to 99.8 wt.% purity *para*-xylene, preferably from 99.0 to 99.8 wt%. Next, *para*-xylene is recovered from the intermediate product or stream to produce a final product or stream having higher purity, *e.g.* at least 99.5 wt.% *para*-xylene and at least greater purity *para*-xylene than that obtained in the first step, by a second step (having two parts) involving: (a) alkylating substantially all of the xylene isomers in the intermediate product or stream with an alkylating agent, while alkylating substantially none of the *para*-xylene; and (b) separating the *para*-xylene in at least 99.5 wt.% purity and in higher purity than that obtained from the first step, from the alkylated xylene isomers to give the final product or stream.

The present invention is distinct from US-A-5,055,630, described earlier, in that the former separates via alkylation whereas the latter uses crystallization. Although it is possible in some prior processes to achieve 99.9 wt.% purity, it is accomplished only with a significant capacity debit. For example, in one conventional process, the increase from producing 99.8 to 99.9 wt.% *para*-xylene results in a 15% reduction in capacity. That is, there is a production capacity/purity trade off which cannot be overcome. Prior commercial processes are separation processes only, whereas the subject process uses separation and reaction processes. In the present invention, one does not have to sacrifice capacity to increase purity.

### Detailed Description of the Invention

It has been discovered that it is not economical nor efficient to produce 99.9+ wt.% *para*-xylene via any single separation process, whether adsorption, crystallization, hydrogen-fluoride extraction, sulfonation or other processes. It has been surprisingly found that the best process arrangement to maximize purity and productivity appears to be attaching an alkylation process at the back end of an adsorption or crystallization process. According to this embodiment the intermediate product is provided as the product resulting from recovery of *para*-xylene from a mixture of xylene isomers using an adsorption or crystallization technique, although other techniques may be used, such as distillation, extraction or sulfonation. Such a hybrid system can easily achieve the desired 99.5+ wt.% purity without any significant productivity loss.

The process of the invention is in essence a purification process, as opposed to the separation processes of the prior art, e.g., US-A-2,801,271. Thus the intermediate product of step (1) preferably contains substantially no components other than para-xylene and its isomers, and step (2) is concerned with purification to *para*-xylene with minimal proportions of other isomers. Looking more to the contrast with the teachings of US-A-2,801,271, that document relates to separation processes rather than purification processes, where separation involves concentrating *para*-xylene from a mixture of paraffins and xylene isomers. Purification in the context of the instant invention concerns purifying a stream of *para*-xylene that is already relatively pure. It is very easy to separate *para*-xylene from paraffins and such separations are well known. However, what is not well known is how to purify *para*-xylene from a mixture of its isomers (rather than separation in bulk from paraffins). Linking together two separation processes does not ipso facto make an effective purification process.

Even with this concept, it is not obvious in which sequence the techniques should be arranged, because once a very high purity is achieved in the first step (e.g., 98 wt%), it is extremely difficult to improve upon this purity for a number of reasons. Undesirable isomerization side reactions occur to convert the *para*-xylene to less desirable *meta*-xylene and/or *ortho*-xylene. Because of the predominant presence of *para*-xylene, it is difficult for the alkylating agent to "see" or come into contact with the isomer impurities to enable them to be alkylated.

According to the invention a two-step process where the selective alkylation is conducted in the second step permits further enriching of the para-xylene stream rather than further degradation so that an ultra-pure *para*-xylene may be obtained.

In the first treatment of the above-mentioned hybrid system, a conventional *para*-xylene recovery technology such as the PAREX® adsorption process or a crystallization process may be used to produce a product stream with 99.0 to 99.8 wt.% *para*-xylene. PAREX is a registered trademark of UOP Inc. These commercial processes are very efficient in such purity range. As explained, what is more difficult is how to upgrade the product purity to 99.5+ wt.% efficiently. Unexpectedly, it was discovered that further *para*-xylene purification is better done via xylene-selective alkylation; the second treatment of the hybrid system of this invention. Preferably an acidic catalyst is used to selectively alkylate just the non-*para*-xylene isomers, selected from ethylbenzene, *meta*-xylene and *ortho*-xylene, while leaving *para*-xylene alone unalkylated. Since the alkylated *para*-xylene isomers boil at substantially higher temperatures compared to unalkylated *para*-xylene, a simple post-alkylation fractionation or a post alkylation carbon adsorption provides efficient removal of the heavies (alkylated products) and produces 99.5+ wt.% *para*-xylene. Moreover, the inventive process does not have appreciable side reactions, reverse reactions, or alkylation of *para*-xylene which decreases yield or conversion to high purity *para*-xylene.

The processes to produce an intermediate product stream having a purity of from about 99.0 to about 99.8 wt.% *para*-xylene, which may be provided in step (1), are well known in the art, though are preferably an adsorption or a crystallization process.

The purification via alkylation process of this invention may be accomplished with either an acidic heterogeneous catalyst, such as proton-exchanged zeolites or an acidic homogeneous catalyst such as aluminum chloride at a temperature in the range of from 10 to 300°C and a pressure from sub-atmospheric to 6.89 MPag (1000 psig), in one embodiment. Preferably, the liquid hourly space velocity (LHSV) ranges from about 0.1 to about 100, and more preferably ranges from about 0.1 to about 10.

The selective alkylation may, in one embodiment, be done in the presence of an acidic catalyst. Preferably, steps are taken so that there is good molecular contact between the catalyst and the xylene isomers which are not *para*-xylene. Without being bound by any one theory, it is hypothesized that the success of the present invention is due at least in part to excellent molecular contact between the catalyst and the non-*para*-xylene isomers. This contact may involve, for example, using a particular high surface area catalyst, extensive mixing or both. Extensive mixing includes, but is not limited to mixing by pressure drop, mixing orifices, static mixers, distribution heads and high speed mixing mechanisms. By "high surface area" catalyst is meant a catalyst having a surface area in excess of about 100 m²/g, preferably in excess of about 200 m²/g, and more preferably one with a surface area in excess of about 300 m²/g. A most preferred high surface area catalyst has a surface area in excess of about 450 m²/g. Additionally, the catalyst is desirably one which does not polymerize the alkylating agent. For example, the catalyst should not be one which substantially dimerizes isobutylene, if this is the alkylation agent.

In one embodiment, the catalyst is a high surface area heterogeneous catalyst, and more preferably may be a high surface area catalyst selected from proton-exchanged zeolites, bentonite clay, acid-treated clay, acidic alumina, solid acids, proton-exchanged resins, and the like. The most preferable catalysts are proton-exchanged zeolites. In another embodiment of the invention, homogeneous acidic catalysts suitable for this invention include, but are not limited to, aluminum chloride, alkyl aluminum chlorides, hydrofluoric acid, and sulfuric acid.

The selective alkylation step of the present invention may be conducted at a temperature in the range of from about 10 to about 300°C, preferably from about 10 to about 150°C and most preferably from about 10 to 50°C. Reaction temperatures greater than about 300°C. are not desired as the tendency for isomerization increases with increasing temperature (the *para*-xylene isomerizes to *ortho*-xylene, *meta*-xylene and/or ethylbenzene), as does the undesired alkylation of *para*-xylene. The alkylation reaction may be conducted in the liquid phase or the vapor phase and thus may be conducted for example at a pressure of from sub-atmospheric to about 6.89 MPag (1000 psig), preferably from sub-atmospheric to 3.45 MPag (500 psig), and most preferably from sub-atmospheric to 2.07 MPag (300 psig). Further, the preferred alkylating agents were found to contain at least one tertiary butyl moiety. In one embodiment, the alkylating agent is selected from the group consisting of isobutylene, tertiary butyl chloride, tertiary butyl benzene, di-tertiary butyl hydroxyl toluene, and isobutylene oligomers. The amount of alkylating agent is dependent on other parameters; for example, it is necessary to have enough alkylating agent to react with all of the impurities. Thus, the amount of alkylating agent should be an effective amount to so alkylate the impurities desired to be removed. A large excess of alkylating agent is preferably avoided.

Under the conditions of preferred embodiments of this invention, the loss of *para*-xylene to alkylation is only in the range of several tenths of one percent. That is, the selectivity of alkylation of non-para-xylene isomers to *para*-xylene may be a surprising 300:1 to 3000:1 ratio. Even a relatively high ratio of 30:1 would generally be unacceptable inasmuch as too much of the *para*-xylene would be lost, as has been established in the prior art. In prior processes, the selectivity was always less than 100:1; the selectivities achievable with this process are surprisingly higher. The selectivity achieved with this invention is unexpected and extraordinary. The process of this invention generally prevents destruction of the major product while selectively removing only the contaminants. This is unusual as previous selective alkylation processes are not this selective, but rather only selectively alkylate the non-*para*-xylene isomers to a much lower extent than is possible in this process. The system of this invention generally and preferably avoids (1) isomerization of the desired *para*-xylene and (2) alkylation of the *para*-xylene (even in the presence of high concentrations thereof), and (3) polymerization of the alkylating agent, *e.g.* dimerization of the isobutylene. Only by avoiding these other reactions are the extraordinarily high selectivities achieved using the inventive process which sets it apart from conventional processes which can only achieve a lower selectivity ratio.

Another advantage of the present invention is that the alkyl, preferably *t*-butyl, -*meta*-xylene and -*ortho*-xylene adducts which may have a lower value will not have a significant effect on the process economics due to the low levels produced. Thus, although some small amount of lower value by-products are produced, the process remains economically attractive.

The invention is illustrated by the following Examples.

### EXAMPLE 1

*Para*-Xylene product from Exxon Chemical PAREX® Adsorption Units (PAU) was provided according to step (1) of the invention. The PAU *para*-xylene contained 99.70 wt.% *para*-xylene, 0.15 wt.% *meta*-xylene, 0.10 wt.% ethylbenzene and 0.05 wt.% *ortho*-xylene. The alkylation catalyst in this test was a proton-exchanged Y zeolite in the form of extrudate (LZY-82, obtained from UOP Inc.). The catalyst was calcined at 300°C under nitrogen prior to testing. The alkylation reactor was a 1.27 cm internal diameter (0.5" I.D.), 7.62 cm (3.0") long stainless steel tubing packed with 2.91 g calcined catalyst. A feed solution containing 99.70 wt.% of the PAU-*para*-xylene as specified above and 0.30 wt.% isobutylene was pumped through the reactor at 1 liquid hourly space velocity (LHSV), ambient temperature and 1.38 MPag (200 psig). Gas chromatograph analysis revealed that the alkylation product contained 0.08 wt.% C₄ compounds, 0.01 wt.% ethylbenzene, 0.05 wt.% *meta*-xylene, 99.36 wt.% *para*-xylene and 0.50 wt.% butylated xylenes and other heavy compounds. *Ortho*-Xylene was completely butylated. After distillation to remove the C₄-materials and the heavies, a *para*-xylene product containing 0.01 wt.% ethylbenzene, 0.05 wt.% *meta*-xylene and 99.94 wt.% *para*-xylene was obtained.

### EXAMPLE 2

This Example was conducted similarly to Example 1. The catalyst was an extrudate of 80 wt.% proton-exchanged ultra stable Y zeolite (VALFOR CP304-37, obtained from The PQ Corp.) and 20 wt.% bentonite clay (obtained from Aldrich Co.). The catalyst was ground to 18/30 mesh and calcined at 300°C prior to loading in the reactor specified in Example 1. A feed solution composed of 99.50 wt.% of the PAU-*para*-xylene of Example 1 and 0.50 wt.% di-tertiary butyl hydroxyl toluene was pumped through the reactor at 0.5 LHSV, ambient temperature and 1.38 MPag (200 psig). The alkylation product contained 0.01 wt.% C4 compounds, 0.01 wt.% ethylbenzene, 0.09 wt.% *meta*-xylene, 99.00 wt.% *para*-xylene, 0.89 wt.% alkylated xylenes and other heavies. There was no *ortho*-xylene detected. After distillation, a *para*-xylene product containing 0.01 wt.% ethylbenzene, 0.09 wt.% *meta*-xylene and 99.90 wt.% *para*-xylene was obtained, as analyzed by gas chromatograph.

### COMPARATIVE EXAMPLE 3

This Example demonstrates the fact that a simple extension of a previously known xylenes-alkylation technique is not suitable for the present purpose of producing 99.9+ wt.% purity *para*-xylene. The Example includes the following two experiments.
(A) Experiment A demonstrates the feasibility of using a conventional alkylation technique for the bulk separation of xylene mixtures. A mixture of 50.00% *para*-xylene and 50.00% *ortho*-xylene was prepared. 3.09 Grams of the mixture was then thoroughly mixed with 3.02 g. of *tertiary-*butyl chloride in a sealed vial. After the mixing, about 0.10 g. of aluminum chloride catalyst was added to the solution. The system was allowed to react at ambient conditions for 30 minutes. A sample of the hydrocarbon phase was taken and analyzed using gas chromatography. It was found that the xylenes fraction of the reaction product contained 97.50 wt.% *para*-xylene and 2.50 wt.% *ortho*-xylene indicating a good separation from the original 50.00% *para*-xylene and 50.00% *ortho*-xylene.
(B) Experiment B demonstrates the failure of using a conventional alkylation technique for producing *para*-xylene in very high purity. 39.91 Grams of a high purity *para*-xylene stream (99.70% *para*-xylene, 0.18% *meta*-xylene, 0.07% ethylbenzene and 0.05% *ortho*-xylene) was mixed with 0.11 g. *tertiary* butyl chloride. 5.00 Grams of the mixture was then brought into contact with 0.46 g. of aluminum chloride catalyst. The mixture was then allowed to react at ambient conditions for 30 minutes. A sample of hydrocarbon phase was analyzed using gas chromatography. The xylenes fraction of the reaction product contained 99.53% *para*-xylene, 0.32% *meta*-xylene, 0.09% *ortho*-xylene and 0.06% ethylbenzene. It is apparent that the alkylation was accompanied by a small amount of side reactions such as *para*-xylene isomerization; note the increase in *meta*-xylene and *ortho*-xylene fractions. As a result, the product had more isomers and *para*-xylene purity decreased. Such low levels of side reactions would not even be detected in bulk separations. But they would be detrimental for purifying high purity products and, as shown, overall *para*-xylene purity would decrease.

### EXAMPLE 4

This Example illustrates the use of a homogeneous catalyst for purifying *para*-xylene. 20.00 Grams of a PAU *para*-xylene product (99.69% *para*-xylene, 0.18% *meta*-xylene, 0.07% ethylbenzene, 0.06% *ortho*-xylene) was thoroughly mixed with 10.16 g. 98% sulfuric acid in a container at ambient temperature and pressure. A mixture of isobutylene and the specified PAU *para*-xylene (0.30% isobutylene and 99.70% PAU *para*-xylene product stream) was placed in a buret and added dropwise to the xylenes/sulfuric acid mixture over a 45 minute period. A high speed magnetic stirrer was used to provide sufficient mixing, i.e., good molecular contact. The hydrocarbon phase was sampled and analyzed by GC at the end of the reaction. It was found that the xylenes product separated out contained 99.75% *para*-xylene, 0.16% *meta*-xylene, 0.06% ethylbenzene and 0.03% *ortho*-xylene. Thus, it is demonstrated that the process of this invention may be successfully performed using a homogeneous catalyst.

### EXAMPLE 5

This Example demonstrates the use of a mordenite zeolite catalyst for purifying *para*-xylene. A mixture of 0.5% isobutylene and 99.5% PAU *para*-xylene product (99.70% *para*-xylene, 0.15% *meta*-xylene, 0.10% ethylbenzene, 0.05% *ortho*-xylene) was prepared. 3.02 Grams of the mixture was brought into contact with 1.09 g. of a mordenite extrudate obtained from UOP Inc. in a sealed bottle at ambient temperature and pressure for 2 hours. According to GC analysis, the xylenes product subsequently separated out contained 99.76% *para*-xylene, 0.15% *meta*-xylene, 0.07% ethylbenzene, 0.03% *ortho*-xylene. It is thus demonstrated that another catalyst may be used in the process of this invention.

## Claims

1. A process for producing at least 99.5 wt % *para*-xylene from a mixture of xylene isomers comprising the steps of:
(1) providing, as an intermediate product, a mixture of xylene isomers containing 99 to 99.8 wt % para-xylene; and
(2) recovering *para*-xylene from the intermediate product to product a final product having para-xylene in a concentration at least 99.5 wt % and greater than the concentration of *para*-xylene in the intermediate product, by a second step which comprises:
(a) alkylating substantially all of the xylene isomers in the intermediate product except *para*-xylene with an alkylating agent having at least one tertiary butyl moiety in the presence of an acidic catalyst and with good molecular contact between the catalyst and the xylene isomers, while alkylating substantially none of the *para*-xylene; and
(b) separating from the alkylated xylene isomers, *para*-xylene in a purity which is at least 99.5 wt % and greater than the concentration of *para*-xylene in the intermediate product stream, to form the final product.

2. The process of Claim 1 wherein the intermediate product is provided as the product resulting from recovery of *para*-xylene from a mixture of xylene isomers using an adsorption, crystallization, distillation, extraction or sulfonation process.

3. The process of Claims 1 or 2 wherein step (2) (a) comprises an extensive mixing technique, optionally selected from pressure drop, mixing orifices, static mixer, distribution head and high speed techniques.

4. The process of any preceding claim where the catalyst is a heterogeneous catalyst having a surface area of at least 100 m²/g, preferably greater than 200 m²/g.

5. The process of Claim 4, wherein the acidic heterogeneous catalyst is selected from the group consisting of proton-exchanged zeolites, bentonite clay, acid-treated clay, acidic alumina, solid acids, proton-exchanged resins and mixtures of any two or more thereof.

6. The process of Claims 1, 2, or 3, wherein the catalyst is a homogeneous catalyst.

7. The process of Claim 6, wherein the catalyst comprises aluminum chloride, an alkyl aluminum chloride, hydrofluoric acid or sulfuric acid.

8. The process of any preceding claim, where the alkylating agent comprises isobutylene, tertiary butyl chloride, tertiary butyl benzene, di-tertiary butyl hydroxyl toluene, an isobutylene oligomer, or a mixture of any two or more thereof.

9. The process of Claim 8, wherein the alkylating agent is isobutylene and the treating step is performed in the presence of a catalyst which does not substantially dimerize the isobutylene.

10. The process of any preceding claim, wherein in step (2) (a) the intermediate product is treated with the alkylating agent at a temperature of from 10 to 300°C.

11. The process of Claim 10, wherein the temperature is from 10 to 50°C.

12. The process of any preceding claim, in which step (2) (a) is performed at a pressure of from sub-atmospheric to 6.89 MPag (1000 psig), preferably to 2.07 MPag (300 psig).

13. The process of any preceding claim when carried out continuously.

14. The process of Claim 13, in which step (2) (a) is conducted at a liquid hourly space velocity (LHSV) of from 0.1 to 100, preferably from 0.1 to 10.

15. The process of any preceding claim, wherein the intermediate product comprises *ortho*-xylene, *meta*-xylene and ethylbenzene.

16. The process of any preceding claim, wherein in step (2) (a) the selectivity of alkylation of non-*para*-xylene isomers to *para*-xylene is from 300:1 to 3000:1.

17. The process of any preceding claim, wherein step (2) (b) comprises fractionation or carbon adsorption.

18. The process of any preceding claim, in which the final product contains *para*-xylene in at least 99.9 wt % purity.

## Patentansprüche

1. Verfahren zur Herstellung von mindestens 99,5 Gew.% *para*-Xylol aus einer Mischung von Xylolisomeren, bei dem in den Stufen
(1) als Zwischenprodukt eine Mischung aus Xylolisomeren bereitgestellt wird, die 99 bis 99,8 Gew.% *para*-Xylol enthält, und
(2) *para*-Xylol in einer zweiten Stufe aus dem Zwischenprodukt gewonnen wird, um ein Endprodukt mit *para*-Xylol in einer Konzentration von mindestens 99,5 Gew.% und höherer *para*-Xylolkonzentration als in dem Zwischenprodukt zu produzieren, wobei in der zweiten Stufe
(a) im wesentlichen die gesamten Xylolisomere in dem Zwischenprodukt außer *para*-Xylol mit einem Alkylierungsmittel mit mindestens einer tertiären Butylgruppe in Gegenwart eines sauren Katalysators und mit gutem molekularen Kontakt zwischen dem Katalysator und den Xylolisomeren alkyliert werden, wobei im wesentlichen kein *para*-Xylol alkyliert wird, und
(b) aus den alkylierten Xylolisomeren *para*-Xylol in einer Reinheit von mindestens 99,5 Gew.% und höherer *para*-Xylolkonzentration als in dem Zwischen-produktstrom abgetrennt wird, um das Endprodukt zu bilden.

2. Verfahren nach Anspruch 1, bei dem das Zwischenprodukt als das Produkt bereitgestellt wird, welches aus der Gewinnung von *para*-Xylol aus einer Mischung von Xylolisomeren unter Verwendung eines Adsorptions-, Kristallisations-, Destillations-, Extraktions- oder Sulfonierungsverfahrens resultiert.

3. Verfahren nach Anspruch 1 oder 2, bei dem Stufe (2) (a) eine umfassende Mischtechnik umfaßt, die gegebenenfalls ausgewählt ist aus Druckminderung, Mischblenden, statischem Mischer, Verteilerkopf und Hochgeschwindigkeitstechniken.

4. Verfahren nach einem der vorhergehenden Ansprüche, bei dem der Katalysator ein heterogener Katalysator mit einer Oberfläche von mindestens 100 m²/g, vorzugsweise mehr als 200 m²/g ist.

5. Verfahren nach Anspruch 4, bei dem der saure heterogene Katalysator ausgewählt ist aus der Gruppe bestehend aus protonenausgetauschten Zeolithen, Bentonit-Ton, säurebehandeltem Ton, saurem Aluminiumoxid, festen Säuren, protonenausgetauschten Harzen und Mischungen aus zwei oder mehreren beliebigen hiervon.

6. Verfahren nach Anspruch 1, 2 oder 3, bei dem der Katalysator ein homogener Katalysator ist.

7. Verfahren nach Anspruch 6, bei dem der Katalysator Aluminiumchlorid, ein Alkylaluminiumchlorid, Fluorwasserstoffsäure oder Schwefelsäure umfaßt.

8. Verfahren nach einem der vorhergehenden Ansprüche, bei dem das Alkylierungsmittel Isobutylen, tert.-Butylchlorid, tert.-Butylbenzol, Di-tert.-butylhydroxyltoluol, ein Isobutylenoligomer oder eine Mischung aus zwei oder mehreren beliebigen hiervon umfaßt.

9. Verfahren nach Anspruch 8, bei dem das Alkylierungsmittel Isobutylen ist und die Behandlungsstufe in Gegenwart eines Katalysators durchgeführt wird, der das Isobutylen nicht wesentlich dimerisiert.

10. Verfahren nach einem der vorhergehenden Ansprüche, bei dem in Stufe (2) (a) das Zwischenprodukt bei einer Temperatur von 10 bis 300°C mit dem Alkylierungsmittel behandelt wird.

11. Verfahren nach Anspruch 10, bei dem die Temperatur 10 bis 50°C beträgt.

12. Verfahren nach einem der vorhergehenden Ansprüche, bei dem Stufe (2) (a) bei einem Druck von subatmosphärischem Druck bis 6,89 MPa Überdruck (1000 psig), vorzugsweise bis 2,07 MPa Überdruck (300 psig) durchgeführt wird.

13. Verfahren nach einem der vorhergehenden Ansprüche, das kontinuierlich durchgeführt wird.

14. Verfahren nach Anspruch 13, bei dem Stufe (2) (a) mit einem stündlichen Flüssigkeitsdurchsatz (LHSV) von 0,1 bis 100, vorzugsweise 0,1 bis 10 durchgeführt wird.

15. Verfahren nach einem der vorhergehenden Ansprüche, bei dem das Zwischenprodukt *ortho*-Xylol, *meta*-Xylol und Ethylbenzol umfaßt.

16. Verfahren nach einem der vorhergehenden Ansprüche, bei dem in Stufe (2) (a) die Selektivität der Alkylierung von nicht*para*-Xylol-Isomeren zu *para*-Xylol 300:1 bis 3000:1 beträgt.

17. Verfahren nach einem der vorhergehenden Ansprüche, bei dem Stufe (2) (b) Fraktionierung oder Kohleadsorption umfaßt.

18. Verfahren nach einem der vorhergehenden Ansprüche, bei dem das Endprodukt *para*-Xylol in mindestens 99,9 Gew.% Reinheit enthält.

## Revendications

1. Procédé pour la production d'au moins 99,5 % en poids de *para*-xylène à partir d'un mélange d'isomères de xylène, comprenant les étapes consistant :
(1) à prendre, comme produit intermédiaire, un mélange d'isomères de xylène contenant 99 à 99,8 % en poids de *para*-xylène ; et
(2) à séparer le *para*-xylène du produit intermédiaire pour obtenir un produit final ayant une concentration de *para*-xylène égale à au moins 99,5 % en poids et supérieure à la concentration de *para*-xylène dans le produit intermédiaire, par une seconde étape qui comprend :
(a) l'alkylation de pratiquement la totalité des isomères de xylène dans le produit intermédiaire, à l'exception du *para*-xylène, avec un agent d'alkylation ayant au moins un groupement tertio-butyle en présence d'un catalyseur acide et avec un contact moléculaire efficace entre le catalyseur et les isomères de xylène, avec une alkylation simultanée pratiquement nulle du para-xylène ; et
(b) l'opération consistant à séparer des isomères de xylène alkylés le para-xylène en une pureté qui est égale à au moins 99,5 % en poids et supérieure à la concentration de para-xylène dans le courant de produit intermédiaire, pour former le produit final.

2. Procédé suivant la revendication 1, dans lequel le produit intermédiaire est formé comme produit résultant de la séparation du *para*-xylène d'un mélange d'isomères de xylène au moyen d'un procédé d'adsorption, de cristallisation, de distillation, d'extraction ou sulfonation.

3. Procédé suivant la revendication 1 ou 2, dans lequel l'étape (2)(a) comprend une technique de mélange poussée, choisie facultativement entre l'utilisation d'une chute de pression, d'orifices de mélange, d'un mélangeur statique, d'une tête de distribution et des techniques de mélange à grande vitesse.

4. Procédé suivant l'une quelconque des revendications précédentes, dans lequel le catalyseur est un catalyseur hétérogène ayant une surface spécifique d'au moins 100 m²/g, de préférence supérieure à 200 m²/g.

5. Procédé suivant la revendication 4, dans lequel le catalyseur hétérogène acide est choisi dans le groupe consistant en zéolites ayant subi un échange de protons, une argile du type bentonite, une argile traitée avec un acide, l'alumine acide, des acides solides, des résines ayant subi un échange de protons et des mélanges de deux ou plus de deux de ces composés.

6. Procédé suivant la revendication 1, 2 ou 3, dans lequel le catalyseur est un catalyseur homogène.

7. Procédé suivant la revendication 6, dans lequel le catalyseur comprend le chlorure d'aluminium, un chlorure d'alkylaluminium, l'acide fluorhydrique ou l'acide sulfurique.

8. Procédé suivant l'une quelconque des revendications précédentes, dans lequel l'agent d'alkylation comprend l'isobutylène, le chlorure de tertio-butyle, le tertio-butylbenzène, le ditertio-butylhydroxytoluène, un oligomère d'isobutylène ou un mélange de deux ou plus de deux quelconques d'entre eux.

9. Procédé suivant la revendication 8, dans lequel l'agent d'alkylation consiste en isobutylène et l'étape de traitement est effectuée en présence d'un catalyseur qui ne provoque pas de dimérisation notable de l'isobutylène.

10. Procédé suivant l'une quelconque des revendications précédentes, dans lequel, dans l'étape (2)(a), le produit intermédiaire est traité avec l'agent d'alkylation à une température de 10 à 300°C.

11. Procédé suivant la revendication 10, dans lequel la température est comprise dans l'intervalle de 10 à 50°C.

12. Procédé suivant l'une quelconque des revendications précédentes, dans lequel l'étape (2) (a) est mise en oeuvre sous une pression allant d'une valeur inférieure à la pression atmosphérique à 6,89 MPa au manomètre (1000 psig), de préférence égale à 2,07 MPa au manomètre (300 psig).

13. Procédé suivant l'une quelconque des revendications précédentes, mis en oeuvre de manière continue.

14. Procédé suivant la revendication 13, dans lequel l'étape (2)(a) est mise en oeuvre à une vitesse spatiale horaire de liquide (VSHL) de 0,1 à 100, de préférence de 0,1 à 10.

15. Procédé suivant l'une quelconque des revendications précédentes, dans lequel le produit intermédiaire comprend l'*ortho*-xylène, le méta-xylène et l'éthylbenzène.

16. Procédé suivant l'une quelconque des revendications précédentes, dans lequel, dans l'étape (2)(a), la sélectivité d'alkylation des isomères, autres que le para-xylène, en *para*-xylène est comprise dans l'intervalle de 300:1 à 3000:1.

17. Procédé suivant l'une quelconque des revendications précédentes, dans lequel l'étape (2)(b) comprend un fractionnement ou une adsorption sur du carbone.

18. Procédé suivant l'une quelconque des revendications précédentes, dans lequel le produit final contient du *para*-xylène à une pureté d'au moins 99,9 % en poids.
